# EUROPEAN PATENT APPLICATION

(11) **EP 2 062 544 A1**
(43) Date of publication of application: **27.05.2009**
(21) Application number: 07806866.5
(22) Date of filing: 06.09.2007
(51) Int. Cl.: A61B 18/12, A61B 18/00

(54) **ENERGY OPERATING DEVICE**

(30) Priority: 11.09.2006 JP 2006245974
(71) Applicant: Olympus Medical Systems Corp., Tokyo 151-0072 (JP)
(72) Inventor: NAKAMOTO, Koji, Tokyo (JP); NAKAMURA, Toshio, Tokyo 192-8512 (JP); TAKAHASHI, Kazuhiko, Tokyo 192-8512 (JP); YOSHIE, Michifumi, Tokyo 192-8512 (JP)
(74) Representative: von Hellfeld, Axel
(86) International application number: PCT/JP2007/067425
(87) International publication number: WO 2008/032639

(57) **Abstract**

An energy surgical device is provided with a treatment section 101 which treats a subject, an energy supply section 103 which supplies energy to the treatment section 101, a state-change detecting section 102 which detects a state change of the treatment section 101, an energy instruction input section 105 which inputs an instruction for the energy supply, and a regulation section 104 which regulates the energy supply by the energy supply section 103 based on the input of the energy supply instruction through the energy instruction input section 105 and a result of detection in the state-change detecting section 102.

## Description

### Technical Field

The present invention relates to an energy surgical device.

### Background Art

In recent years, an energy surgical device such as an electric knife, which utilizes high frequencies (hundreds of kilohertz) and high voltages (hundreds to thousands of volts), is widely used as treatment means for a surgical operation and the like. Jpn. Pat. Appln. KOKAI Publication No. 7-8503 is an example of a patent document that discloses a surgical device using high-frequency energy.

Further, an ultrasonic treatment device, which resects or coagulates an organism by sucking or grasping the organism and applying ultrasonic vibration to a sucking or grasping member, is widely used as treatment means for a surgical operation and the like. Jpn. Pat. Appln. KOKAI Publication No. 10-5236 is an example of a patent document that discloses one such ultrasonic treatment device.

### Disclosure of Invention

In the conventional energy surgical device described above, the organism may sometimes be excessively incised or perforated if an energy supply section supplies energy to a treatment section that applies excessive force to the organism. Therefore, a doctor who handles the energy surgical device must perform a treatment most carefully lest excessive force be applied to the distal end portion of the energy surgical device.

The present invention has been made in consideration of these circumstances, and its object is to provide an energy surgical device with higher safety, capable of preventing excessive incision or perforation of an organism.

In order to obtain the above object, according to a first aspect, there is provided an energy surgical device aspect comprising:
a treatment section which treats a subject;
an energy supply section which supplies energy to the treatment section;
a state-change detecting section which detects a state change of the treatment section;
an energy instruction input section through which an instruction for the energy supply is input; and
a regulation section which regulates the energy supply by the energy supply section based on the input of the energy supply instruction through the energy instruction input section and a result of detection in the state-change detecting section.

According to a second aspect, there is provided an energy surgical device comprising:
a treatment section which treats a subject;
an energy supply section which supplies energy to the treatment section;
a force amount change detecting section which detects a change of an amount of force received by the treatment section;
an energy instruction input section which inputs an instruction for the energy supply; and
a regulation section which regulates the energy supply by the energy supply section based on the input of the energy supply instruction through the energy instruction input section and a result of detection in the state-change detecting section.

According to a third aspect, there is provided an energy surgical device comprising:
a treatment section which treats a subject;
an energy supply section which supplies energy to the treatment section;
a state-change detecting section which detects a state change of the treatment section;
an energy instruction input section which inputs an instruction for the energy supply;
a power section which actively drives the treatment section;
a power instruction input section which instructs the power section to operate;
a control section which controls the power section in accordance with the operation instruction through the power instruction input section; and
a regulation section which regulates the energy supply by the energy supply section and/or the drive by the power section based on the input of the power instruction through the power instruction input section and a result of detection in the state-change detecting section.

### Brief Description of Drawings

FIG. 1 is a diagram for illustrating an outline of a first embodiment;
FIG. 2A is a view showing a configuration of the first embodiment of the present invention;
FIG. 2B is a view showing a configuration of the first embodiment;
FIG. 3 is a sectional view of a portion A-A of an electric knife 3 for flexible scope to which a strain gauge 8 is affixed;
FIG. 4 is a diagram showing an internal configuration of an electrical surgical device 1;
FIG. 5 is a flowchart for illustrating the operation of the configuration of the first embodiment;
FIG. 6 is a view for illustrating the details of a case where the first embodiment is applied to ESD;
FIG. 7A is a view showing a case where three strain gauges 8a, 8b and 8c are used;
FIG. 7B is a view showing a case where two strain gauges 8a and 8b are used;
FIG. 8 is a diagram for illustrating an outline of a second embodiment;
FIG. 9 is a view showing a configuration of the second embodiment of the present invention;
FIG. 10 is a general view showing the way an electrically-driven bendable knife 40 for flexible scope is inserted into a flexible endoscope 19;
FIG. 11 is a diagram showing an internal configuration of an electrical surgical device 1;
FIG. 12 is a flowchart for illustrating the operation of the configuration of the second embodiment;
FIG. 13 is a view showing a configuration of a third embodiment of the present invention;
FIG. 14 is a diagram showing an internal configuration of an electrical surgical device 1;
FIG. 15 is a sectional view of a human abdomen 53 under an endoscopic surgical operation;
FIG. 16 is a view showing a configuration of a fourth embodiment of the present invention;
FIG. 17 is a diagram showing an internal configuration of an electrical surgical device 1; and
FIG. 18 is a view for illustrating the details of a case where the fourth embodiment is applied to an endoscopic surgical operation.

### Best Mode for Carrying Out the Invention

Embodiments of the present invention will now be described with reference to the drawings.

### (First Embodiment)

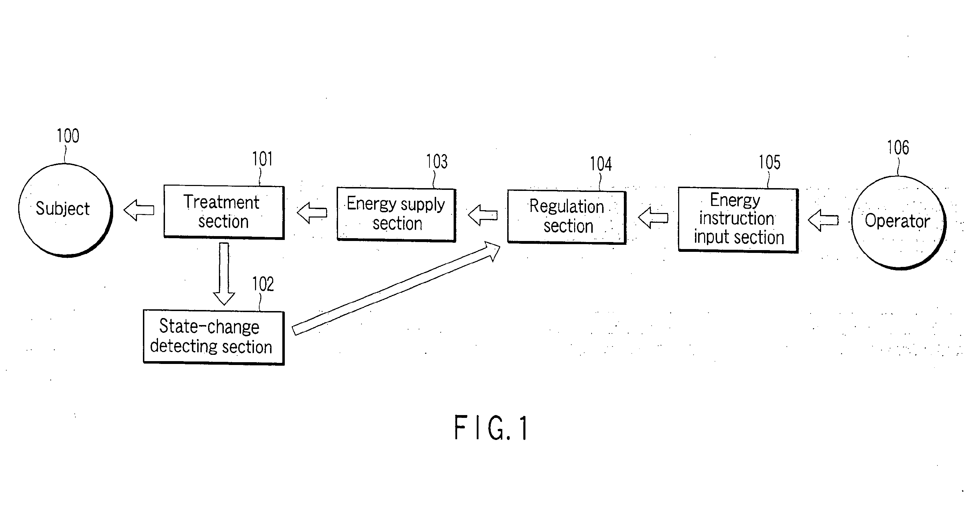

A first embodiment of the present invention will now be described with reference to FIGS. 1 to 7B. An energy surgical device of the present embodiment is provided with a state-change detecting section and regulation section. The state-change detecting section detects a state change of an electric knife for use as a treatment section, e.g., a force that acts on the electric knife. The regulation section regulates the amount of energy supply to the treatment section, based on a result of detection by the state-change detecting section. This configuration serves to regulate the throughput of the treatment section that treats a subject.

FIG. 1 is a diagram for illustrating an outline of the first embodiment. In FIG. 1, an energy instruction input section 105 receives an instruction for energy supply to a treatment section 101 from an operator. The energy instruction input section 105 transmits this instruction input signal to a regulation section 104.

On the other hand, a state-change detecting section 102 detects a state change of the treatment section 101. The detected state change is transmitted to the regulation section 104. Based on a signal from the energy instruction input section 105 and a signal from the state-change detecting section 102, the regulation section 104 determines and outputs an output signal to an energy supply section 103. In response to the signal from the regulation section 104, the energy supply section 103 supplies energy to the treatment section 101. On receipt of the energy from the energy supply section 103, the treatment section 101 treats a subject 100.

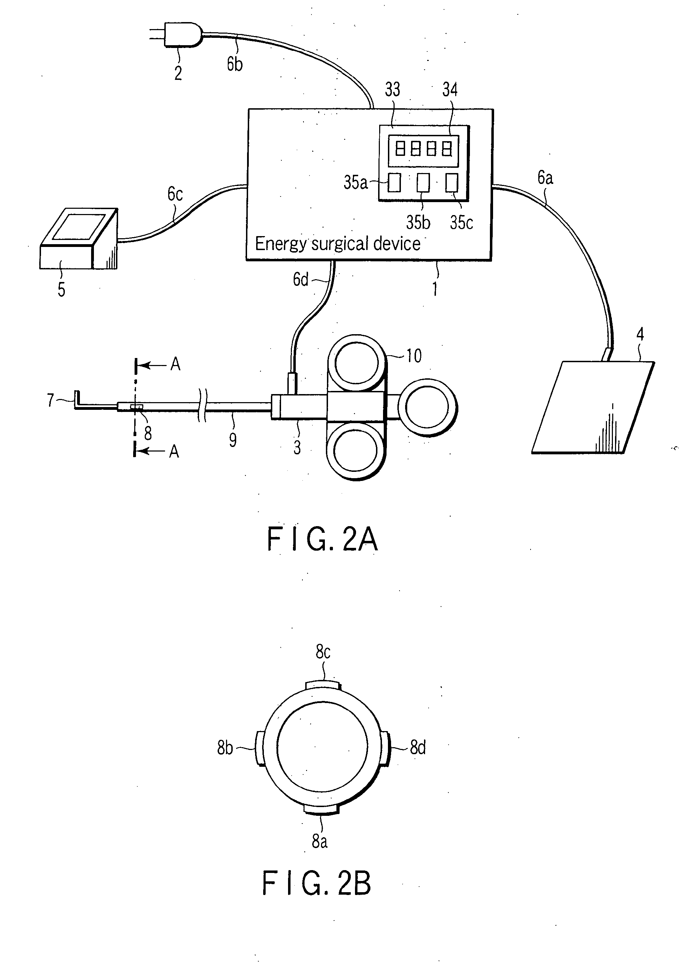

FIG. 2A is a view showing a configuration of the first embodiment of the present invention. The electrical surgical device 1 is connected with a power cord 2, an electric knife 3 for flexible scope for treating a patient based on a high-frequency signal output from the electrical surgical device 1, a counterelectrode plate 4 to be attached to the patient's foot, back, or the like, and a foot switch 5 for input of a high-frequency output instruction, by cords 6a to 6d, individually. The electrical surgical device 1 is provided with a threshold display section 34 and threshold input section 33, which is composed of threshold setting buttons 35a to 35c.

The distal end portion of the electric knife 3 for flexible scope is provided with an electrode section 7. The material of the electrode section 7 is based on SUS304, which conducts electricity. Further, a strain gauge 8 is affixed to the electric knife 3 for flexible scope, and it can detect forces that act on the distal end portion of the electric knife 3 for flexible scope.

FIG. 2B is a sectional view of a portion A-A of the electric knife 3 for flexible scope to which the strain gauge 8 is affixed. The strain gauge 8 is affixed in a plurality of directions (8a to 8d) to the electric knife 3 for flexible scope so that it can detect forces that act on the electric knife 3 for flexible scope in a plurality of directions. Further, strain gauge 8 is connected to a sensor signal processor (mentioned later) in the electrical surgical device 1 by a cable (not shown). An insulating cover 9 is formed of an insulator, which prevents an electric leakage when a voltage is applied to the electrode section 7. A handle 10 is movable in association with the electrode section 7. The operator controls the projection and retraction of the electrode section 7 by moving this handle 10.

The electric knife 3 for flexible scope is combined with a flexible endoscope 19 to perform a treatment.

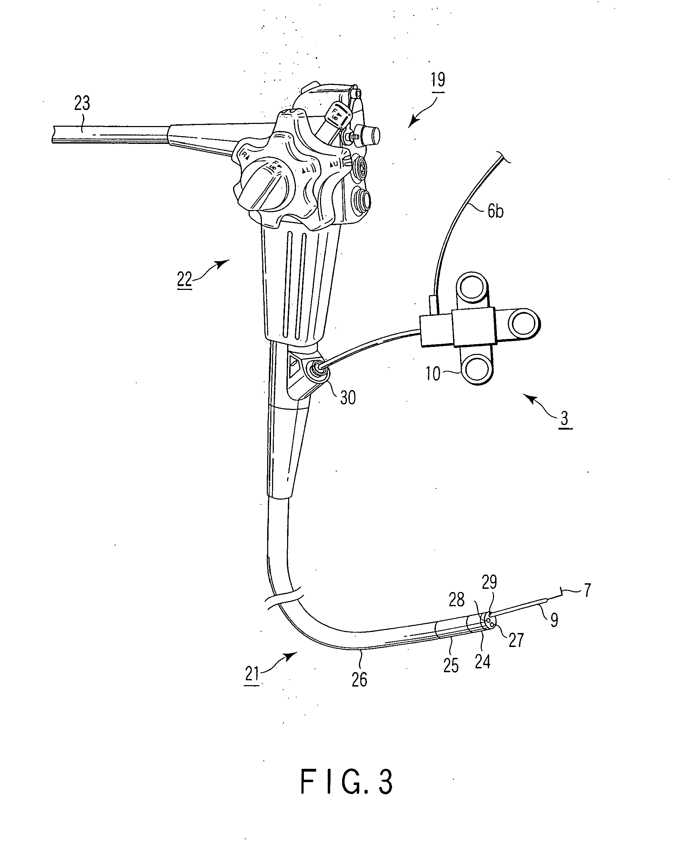

FIG. 3 is a general view showing the way the electric knife 3 for flexible scope is inserted into the flexible endoscope 19. The flexible endoscope 19 includes a flexible insertion section 21 and hand-held operating section 22, and the operating section 22 is coupled with a universal cord 23 to be connected to a light source unit, video processor (not shown), etc. Further, the operating section 22 is formed with a channel tube proximal opening 30 into which a treatment tool is inserted. The flexible endoscope 19 contains an observation optical system, an illumination optical system, a channel tube, etc.

The insertion section 21 is composed of an endoscope tip portion 24, an endoscope bending section 25 adjacent thereto, and an endoscope flexible tube 26 connected to the hand side of the scooping section 25. Further, the distal end portion of the insertion section 21 is formed with an observation window 27 of the observation optical system, an illumination window 28 of the illumination optical system, and a channel tube distal opening 29. If the electric knife 3 for flexible scope is inserted into the channel tube proximal opening 30 with its distal end forward, the distal end of the electric knife 3 for flexible scope projects from the channel tube distal opening 29.

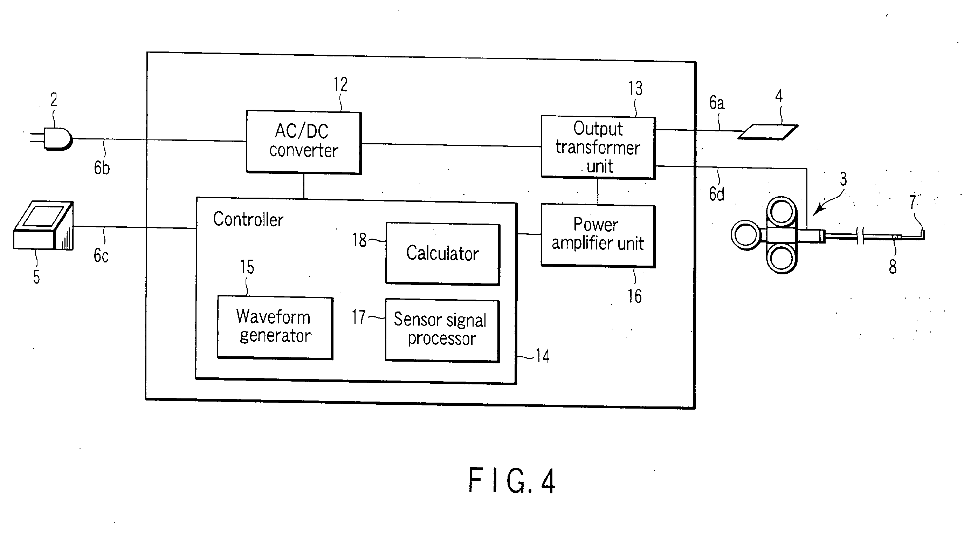

FIG. 4 is a diagram showing an internal configuration of the electrical surgical device 1. An AC/DC converter 12 generates a DC voltage based on a commercial power supply through the power cord 2 and supplies the DC voltage to component devices in the electrical surgical device 1. An output transformer unit 13 applies a voltage to the electrode section 7 and counterelectrode plate 4. A current is supplied with the counterelectrode plate 4 attached to the patient's back, foot, or the like so that the electrode section 7 contacts the patient's lesion. While regions near the counterelectrode plate 4 and electrode section 7 are both heated, the temperature of the counterelectrode plate 4, which has a wide area, rises little. Since the electrode section 7 has a small area, on the other hand, the temperature of the region near the electrode section 7 drastically increases, thereby causing an organism to be cauterized.

A controller 14 for controlling various parts includes a waveform generator 15, sensor signal processor 17, and calculator 18. The waveform generator 15 generates a waveform for a high-frequency treatment. A power amplifier unit 16 amplifies the energy of the waveform generated in the waveform generator 15. The sensor signal processor 17 processes a sensing signal from the strain gauge 8 and detects a force that acts on the distal end portion of the electric knife 3 for flexible scope.

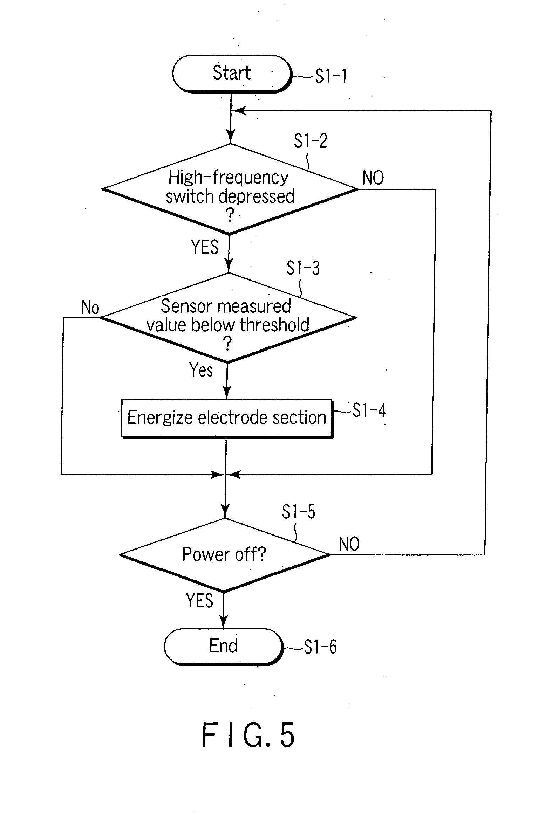

The operation of the aforementioned configuration of the first embodiment will now be described with reference to the flowchart of FIG. 5. Processing starts at Step S1-1. If a signal from the foot switch (high-frequency switch) 5 is input as a conduction instruction in Step S1-2, the program proceeds to Step S1-3.

While the strain gauge 8 detects the force that acts on the distal end portion of the electric knife 3 for flexible scope, the resulting sensing information is delivered to the controller 14. A threshold value for determining from the sensing information whether or not the force on the distal end portion of the electric knife 3 for flexible scope is excessive is previously set in the controller 14. This threshold value can be separately set to an arbitrary value by means of the threshold setting buttons 35a to 35c in the threshold input section 33 and displayed on the threshold display section 34. Since the threshold value can be set to an arbitrary value, the operator can set any desired threshold value or a threshold value corresponding to the conditions of the lesion tissue.

The sensor signal processor 17 of the controller 14 obtains the sensing information by processing the sensing signal from the strain gauge 8. The controller 14 determines whether or not the sensing information obtained in the sensor signal processor 17 is not above the threshold value (Step S1-3). If the sensing information not higher, the program proceeds to Step S1-4.

In Step S1-4, the controller 14 outputs the waveform generated in the waveform generator 15 to the power amplifier unit 16. The power amplifier unit 16 amplifies the energy of the waveform received from the controller 14 and outputs it to the output transformer unit 13. In response to a voltage obtained from the power amplifier unit 16, the output transformer unit 13 delivers a high-frequency output to the electrode section 7 and counterelectrode plate 4. Since the counterelectrode plate 4 and electrode section 7 are attached to the patient's foot or the like and the vicinity of a lesioned region, respectively, a current flows through the body of the patient (subject 100). If the current conduction occurs, the lesioned region is cauterized as a result. After the current conduction, the program proceeds to Step S1-5.

If the operator does not disconnect the electrical surgical device 1 from the power supply in Step S1-2, the program returns to Step S1-2. The region near the lesioned region is cauterized by repeating Steps S1-2 to S1-5 (loop control).

If the signal as the conduction instruction from the foot switch 5 is not input in Step S1-2, on the other hand, the program proceeds to Step S1-5. If the decision is then NO, the program returns to Step S2. Steps S1-2 and S1-5 are repeated (loop control) in a standby state until the operator turns off the power or depresses the foot switch 5.

If the sensing information obtained in the sensor signal processor 17 exceeds the threshold value in Step S1-3, on the other hand, Steps S1-2, S1-3 and S1-5 are executed. If the decision in Step S1-5 is NO, the program returns to Step S2. Steps S1-2, S1-3 and S1-5 are repeated (loop control) in a standby state until the operator turns off the power or depresses the foot switch 5. In this case, no current conduction is performed even when the operator depresses the foot switch 5.

If the operator disconnects the electrical surgical device 1 from the power supply in Step S1-5, the program proceeds to Step S1-6, whereupon the processing terminates.

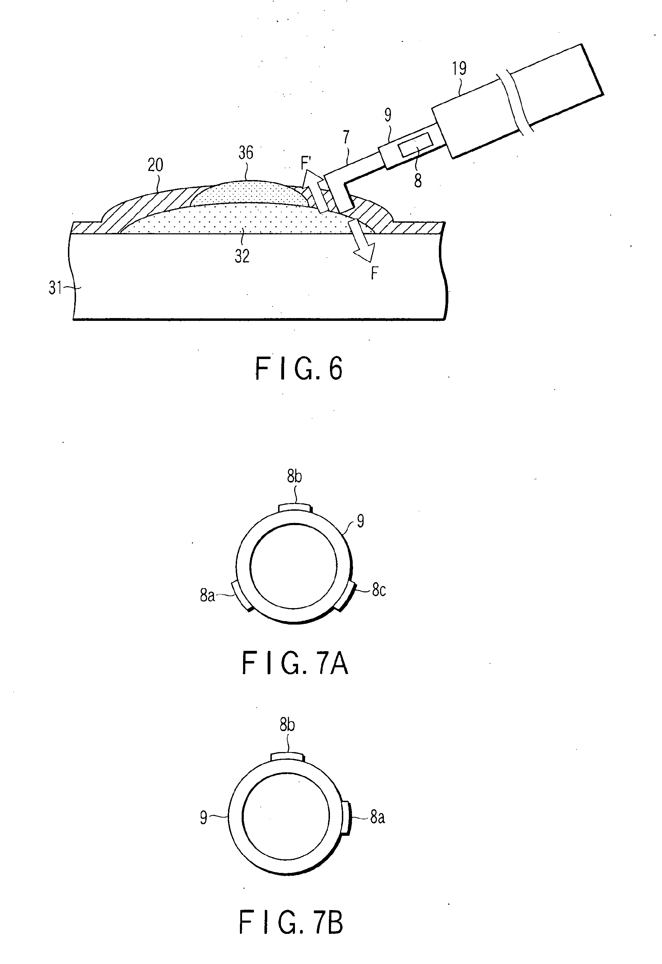

A case where the foregoing first embodiment is applied to the endoscopic submucosal dissection (hereinafter referred to as ESD) will now be described in detail with reference to FIG. 6. The ESD is a procedure that uses an endoscope to collectively resect a lesion in the stomach or large intestine. FIG. 6 is a schematic view of a lesion in a stomach. In carrying out the ESD, a saline is locally injected in advance between a proper muscle layer 31 and mucosal tissue 20 that includes a lesioned region 36. The electric knife 3 for flexible scope to be used is inserted through the channel tube proximal opening 30 of the flexible endoscope 19. The operator operates the flexible endoscope 19 so that the electrode section 7 contacts the mucosal tissue 20. By applying a high-frequency voltage to the electrode section 7 in this state, the operator gradually cauterizes and incises the mucosal tissue 20 around the lesioned region 36.

As shown in the drawing, a force F from the electrode section 7 acts on the mucosal tissue 20, while a repulsion F' from the mucosal tissue 20 acts on the electrode section 7. A relational expression F = F' holds for the force F and repulsion F'. If the high-frequency voltage is applied with the force F to an excessively high degree, the mucosal tissue 20 may be incised beyond necessity or the proper muscle layer 31 may be perforated.

In the first embodiment, therefore, the application of the high-frequency voltage to the electrode section 7 is controlled if the repulsion F' detected by the strain gauge 8 is above a threshold value. Thus, if excessive force is applied to the electrode section 7, excessive incision of the mucosal tissue 20 and perforation of the proper muscle layer 31 can be prevented to improve the safety.

According to the present embodiment, the ESD in the stomach is given as an example of the procedure. If the principle described in connection with the present embodiment is used, however, excessive incision or perforation of the organism can be prevented to improve the safety when excessive force is applied to the electrode section 7 in any of procedures that utilize other flexible endoscopes and energy surgical devices.

The following is a description of the correspondence between the individual devices of the first embodiment and the processing sections shown in FIG. 1. The foot switch 5 corresponds to the energy instruction input section 105. The controller 14 corresponds to the regulation section 104. The output transformer unit 13 corresponds to the energy supply section 103. The electric knife 3 for flexible scope corresponds to the treatment section 101. The strain gauge 8 corresponds to the state-change detecting section 102.

Although the electrode section 7 used in this case is of a hook type, its shape is not specially limited. An unbent or insertable shape may be used instead.

### (Second Embodiment)

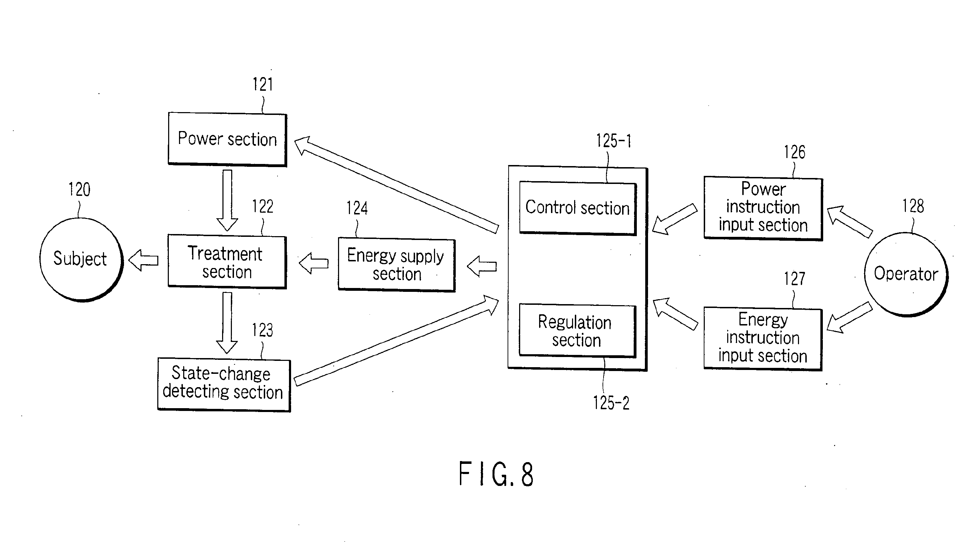

A second embodiment of the present invention will now be described mainly with reference to FIGS. 8 to 12.

FIG. 8 is a diagram for illustrating an outline of the second embodiment. In FIG. 8, a power instruction input section 126 receives an instruction for operating a treatment section 122 from an operator 128. Further, an energy instruction input section 127 receives an instruction for supplying energy to the treatment section 122 from the operator 128. The power instruction input section 126 transmits a signal associated with the power instruction to a control section 125-1. The energy instruction input section 127 transmits an instruction associated with the energy instruction to a regulation section 125-2.

Further, a state-change detecting section 123 transmits a state change of the treatment section 122 to the regulation section 125-2. The control section 125-1 determines an output to a power section 121, based on the signal from the power instruction input section 126 and a signal from the state-change detecting section 123, and delivers an output signal. Furthermore, the regulation section 125-2 determines an output to an energy supply section 124, based on a signal from the energy instruction input section 127 and a signal from the state-change detecting section 123, and delivers an output signal. The outputs to the energy supply section 124 and power section 121 may be determined alternatively.

The power section 121 drives the treatment section 122 based on the signal from the control section 125-1. Further, the energy supply section 124 supplies energy to the treatment section 122 based on the signal from the regulation section 125-2. The treatment section 122 treats a subject 120 on receipt of the energy from the energy supply section 124.

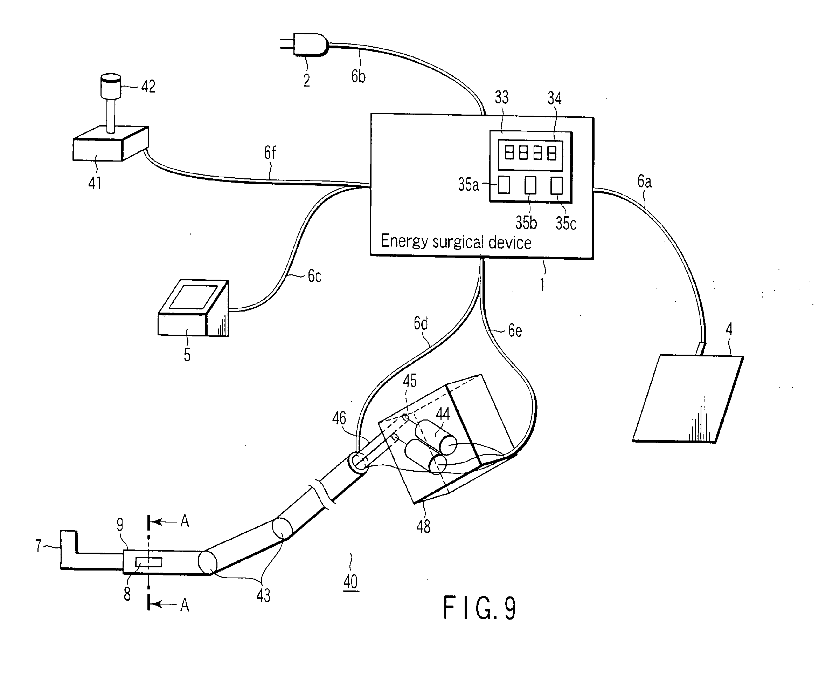

FIG. 9 is a view showing a configuration of the second embodiment of the present invention. An electrical surgical device 1 is connected with a power cord 2, an electrically-driven bendable knife 40 for flexible scope for treating a patient based on a high-frequency signal output from the electrical surgical device 1, a counterelectrode plate 4 to be attached to the patient's foot, back, or the like, a foot switch 5 for input of a high-frequency output instruction, and a bending instruction input unit 44 for input of a bending instruction for the electrically-driven bendable knife 40 for flexible scope, by cords 6a to 6f, individually.

The electrical surgical device 1 is provided with a threshold display section 34 and threshold input section 33, which is composed of threshold setting buttons 35a to 35c. The distal end portion of the electrically-driven bendable knife 40 for flexible scope is provided with an electrode section 7. The material of the electrode section 7 is based on SUS304, which conducts electricity. Further, a strain gauge 8 is affixed to the electrically-driven bendable knife 40 for flexible scope, and it can detect forces that act on the distal end portion of the electrically-driven bendable knife 40 for flexible scope.

FIG. 2B is a sectional view of a portion A-A of the electrically-driven bendable knife 40 for flexible scope to which the strain gauge 8 is affixed. The strain gauge 8 is affixed in a plurality of directions (8a to 8d) to the electric knife 3 for flexible scope so that it can detect forces that act on the electric knife 3 for flexible scope in a plurality of directions. Further, the strain gauge 8 is connected to a sensor signal processor (mentioned later) in the electrical surgical device 1 by a cable (not shown). An insulating cover 9 is formed of an insulator, which prevents an electric leakage when a voltage is applied to the electrode section 7.

The electrically-driven bendable knife 40 for flexible scope includes bending sections 43 that are bendable. The bending sections 43 can be bent by pulling a wire 46. The wire 46 is pulled by a pulley 45 as an electromagnetic motor 44 rotates. An encoder (not shown) is incorporated in the electromagnetic motor 44. This encoder can detect a rotational frequency and transmits the rotational frequency to a motor controller (mentioned later) through a cord 6e. The electromagnetic motor 44 and pulley 45 are incorporated in a motor box 48. The bending instruction is input to the bending sections 43 by operating a joystick 42 on the bending instruction input unit 41.

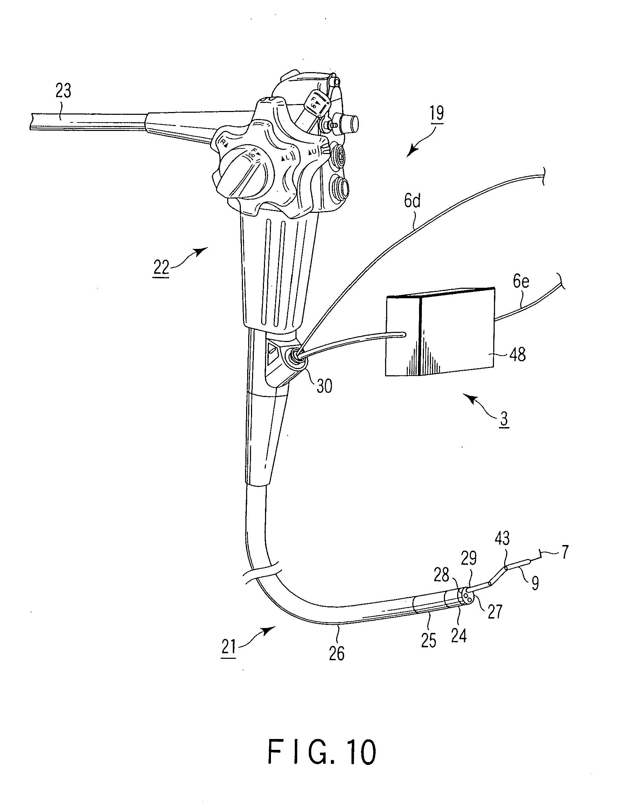

The electrically-driven bendable knife 40 for flexible scope is combined with a flexible endoscope 19 to perform a treatment. FIG. 10 is a general view showing the way the electrically-driven bendable knife 40 for flexible scope is inserted into the flexible endoscope 19. The basic configuration of the flexible endoscope 19 of the present embodiment is the same as the substance described in connection with the first embodiment.

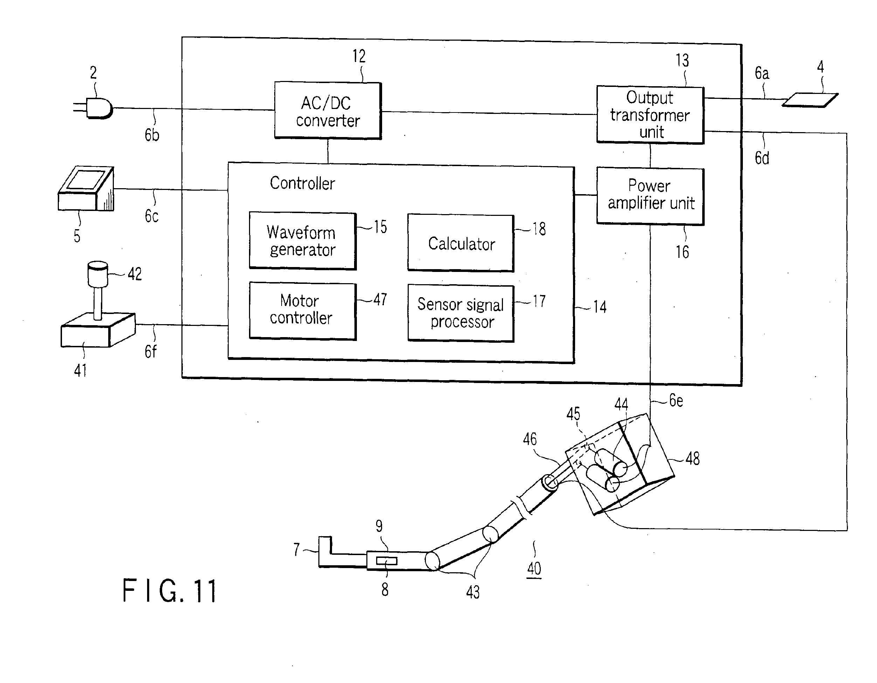

FIG. 11 is a diagram showing an internal configuration of the electrical surgical device 1. An AC/DC converter 12 generates a DC voltage based on a commercial power supply through the power cord 2 and supplies the DC voltage to component devices in the electrical surgical device 1. An output transformer unit 13 applies a voltage to the electrode section 7 and counterelectrode plate 4. A current is supplied with the counterelectrode plate 4 attached to the patient's back, foot, or the like so that the electrode section 7 contacts the patient's lesion. While regions near the counterelectrode plate 4 and electrode section 7 are both heated, the temperature of the counterelectrode plate 4, which has a wide area, rises little. Since the electrode section 7 has a small area, on the other hand, the temperature of the region near the electrode section 7 drastically increases, thereby causing an organism to be cauterized.

A controller 14 for controlling various parts includes a waveform generator 15, sensor signal processor 17, calculator 18, and motor controller 47. The waveform generator 15 generates a waveform for a high-frequency treatment. A power amplifier unit 16 amplifies the energy of the waveform generated in the waveform generator 15 and the energy of a control signal generated in the motor controller 47.

The sensor signal processor 17 processes a signal from the strain gauge 8 and detects a force that acts on the distal end portion of the electrically-driven bendable knife 40 for flexible scope. The motor controller 47 calculates a signal for controlling the electromagnetic motor 44.

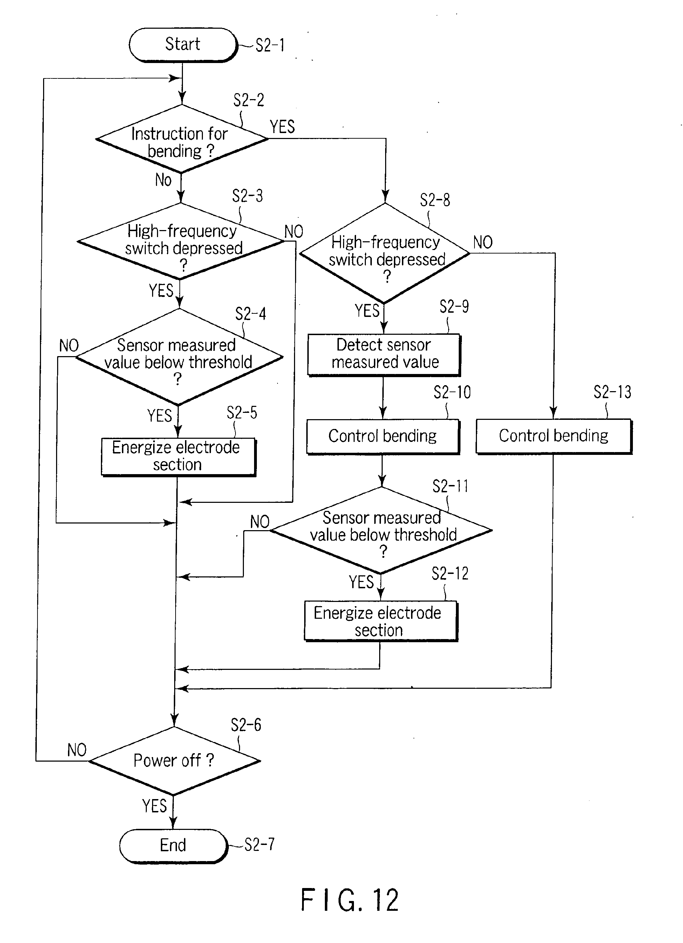

The operation of the aforementioned configuration of the second embodiment will now be described with reference to the flowchart of FIG. 12. A case where the bending instruction is not given in Step S2-2 will be described first. Processing starts at Step S2-1. If no signal is input as the bending instruction from the joystick 42 in Step S2-2, the program proceeds to Step S2-3. If a signal from the foot switch (high-frequency switch) 5 is input as a conduction instruction in Step S2-3, the program proceeds to Step S2-4.

While the strain gauge 8 detects the force that acts on the distal end portion of the electrically-driven bendable knife 40 for flexible scope, the resulting sensing information is delivered to the controller 14. A threshold value for determining from the sensing information whether or not the force on the distal end portion of the electrically-driven bendable knife 40 for flexible scope is excessive is previously set in the controller 14. The threshold value is adjusted to a value such that a mucous membrane is instantaneously resected when a force above it acts on the electrically-driven bendable knife 40 for flexible scope. The threshold value can be separately set to an arbitrary value by means of the threshold setting buttons 35a to 35c in the threshold input section 33 and displayed on the threshold display section 34. Since the threshold value can be set to an arbitrary value, the operator can set any desired threshold value or a threshold value corresponding to the conditions of the lesion tissue.

The sensor signal processor 17 of the controller 14 obtains the sensing information by processing the sensing signal from the strain gauge 8. The controller 14 determines whether or not the sensing information obtained in the sensor signal processor 17 is not above the threshold value (Step S2-4). If the sensing information is not higher, the program proceeds to Step S2-5.

In Step S2-5, the controller 14 outputs the waveform in the waveform generator 15 to the power amplifier unit 16. The power amplifier unit 16 amplifies the energy of the waveform received from the controller 14 and outputs it to the output transformer unit 13. In response to a voltage obtained from the power amplifier unit 16, the output transformer unit 13 applies a voltage between the electrode section 7 and counterelectrode plate 4. Since the counterelectrode plate 4 and electrode section 7 are attached to the patient's foot or the like and the vicinity of a lesioned region 36, respectively, a current flows through the body of the patient. If the current conduction occurs, the lesioned region is cauterized as a result. After the current conduction, the program proceeds to Step S2-6.

If the operator does not disconnect the electrical surgical device 1 from the power supply in Step S2-6, the program returns to Step S2-2. The region near the lesioned region is cauterized by repeating Steps S2-2 to S2-6 (loop control).

If the operator does not depress the foot switch 5 for the conduction instruction in Step S2-3, the program proceeds to Step S2-6. If the decision is then NO, the program returns to Step S2-2. Steps S2-2, S2-3 and S2-6 are repeated (loop control) in a standby state until the operator turns off the power or depresses the foot switch 5.

If the sensing information obtained in the sensor signal processor 17 exceeds the threshold value in Step S2-4, Steps S2-2, S2-3, S2-4 and S2-6 are executed. If the decision in Step S2-6 is NO, the program returns to Step S2-2. Steps S2-2, S2-3, S2-4 and S2-6 are repeated (loop control) in a standby state until the operator turns off the power or depresses the foot switch 5. In this case, no current conduction is performed even when the operator depresses the foot switch 5.

If the operator disconnects the electrical surgical device 1 from the power supply in Step S2-6, the program proceeds to Step S2-7, whereupon the processing terminates.

The following is a description of a case where the bending instruction is given in Step S2-2. Processing starts at Step S2-1. If a signal is input as the bending instruction from the joystick 42 in Step S2-2, the program proceeds to Step S2-8. If a signal from the foot switch 5 is input as a conduction instruction in Step S2-8, the program proceeds to Step S2-9. In Step S2-9, the sensor signal processor 17 processes the signal from the strain gauge 8 and detects the force that acts on the distal end portion of the electrically-driven bendable knife 40 for flexible scope, whereupon the program proceeds to Step S2-9. In Step S2-10, the bending sections 43 are controlled in bending lest the sensing information exceed the threshold value, based on the bending instruction information obtained in Step S2-2 and the sensing information obtained in Step S2-9, whereupon the program proceeds to Step S2-11.

The same processing as Step S2-4 is performed in Step S2-11. If the sensing information is below the threshold value, the program proceeds to Step S2-12. The same processing as Step S2-5 is performed in Step S2-12, that is, the electrode section 7 is energized to cauterize the lesioned region. If the decision in Step S2-6 is NO, the program returns to Step S2-2. Steps S2-2, S2-8 to S2-12, and S2-6 are repeated (loop control) in a standby state until the operator turns off the power or depresses the foot switch 5.

If the signal as the conduction instruction from the foot switch 5 is not input in Step S2-8, moreover, the program proceeds to Step S2-13, where bending control is performed according to the bending instruction obtained in Step S2-2. Thereafter, the program proceeds to Step S2-6. Steps S2-2, S2-8, S2-13 and S2-6 are repeated (loop control) in a standby state until the operator turns off the power or depresses the foot switch 5.

If the sensing information exceeds the threshold value in Step S2-11, moreover, the program proceeds to Step S2-6. Steps S2-2, S2-8, S2-9, S2-10, S2-11 and S2-6 are repeated (loop control) in a standby state until the operator turns off the power or depresses the foot switch 5. In consequence, a sensor measured value does not fall below the threshold value even when the bending section 47 is controlled in bending, so that no conduction is performed.

A case where the foregoing second embodiment is applied to the endoscopic submucosal dissection (hereinafter referred to as ESD) will now be described in detail with reference to FIG. 6. A description of common portions that are shared with the first embodiment is omitted, and only differences from the first embodiment are explained herein. In the second embodiment, the bending sections 43 are controlled in bending so that F is smaller if a repulsion F' detected by the strain gauge 8 is above a threshold value. If F is above the threshold value after the bending control, the voltage applied to the electrode section 7 is controlled. Thus, if excessive force is applied to the electrode section 7, excessive incision of the mucosal tissue 20 and perforation of the proper muscle layer 31 can be prevented to improve the safety.

According to the present embodiment, the ESD in the stomach is given as an example of the procedure. If the principle described in connection with the present embodiment is used, however, excessive incision or perforation of the organism can be prevented to improve the safety when excessive force is applied to the electrode section 7 in any of procedures that utilize other flexible endoscopes and energy surgical devices.

The following is a description of the correspondence between the individual devices of the second embodiment and the processing sections shown in FIG. 8. The bending instruction input unit 41 corresponds to the power instruction input section 126. The foot switch 5 corresponds to the energy instruction input section 127. The controller 14 corresponds to the control section 125-1 and regulation section 125-2. The output transformer unit 13 corresponds to the energy supply section 124. The electrically-driven bendable knife 40 for flexible scope corresponds to the power section 121 and treatment section 122. The strain gauge 8 corresponds to the state-change detecting section 123.

Although the wire 46 and electromagnetic motor 44 are used as means for bending the bending sections 43 according to the second embodiment, they may be successfully replaced with any other means that include a bending mechanism. For example, a pneumatic actuator may be substituted for the electromagnetic motor 44, and an artificial muscle may be used in place of the wire 46 and electromagnetic motor 44.

Although the smoothly bendable bending sections 43 are used as means for operating the electrode section 7, moreover, it may be replaced with any means that can operate the electrode section 7. For example, an angling mechanism or a rotation mechanism may be used instead.

Although the joystick 41 is used as a bending instruction section, furthermore, the form of an input method is not specially limited in the present embodiment. For example, a haptic device, a touch-panel monitor, or voice recognition may be used instead.

Although the bending of the bending sections 43 and the high-frequency signal are controlled, moreover, only one of them may be controlled. In the case where only one is controlled, an input interface may be provided that allows the operator to select the controlled object.

Although the electrode section 7 is of a hook type, furthermore, its shape is not specially limited. An unbent or insertable shape may be used instead.

### (Third Embodiment)

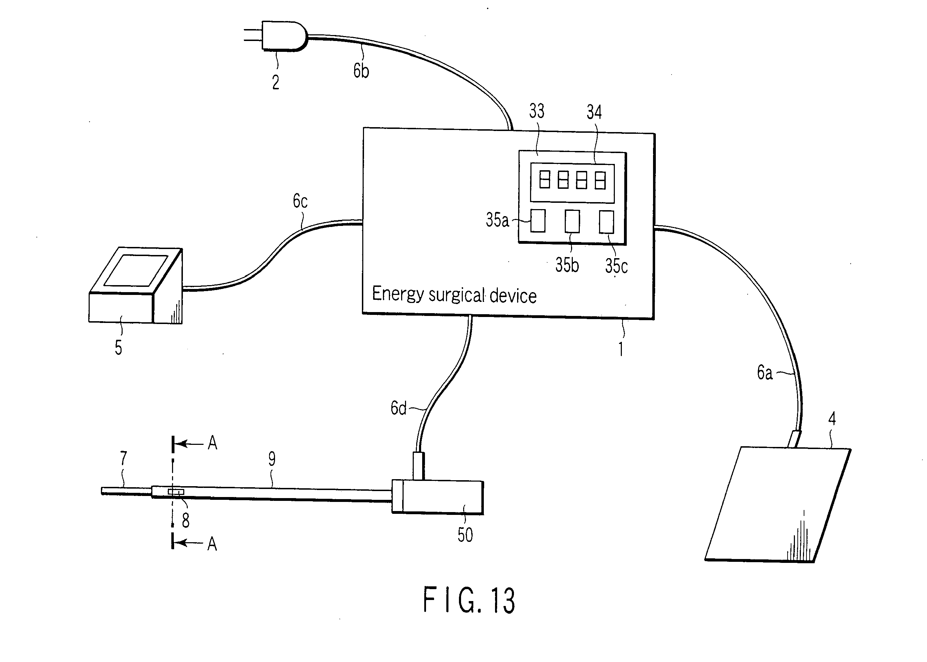

A third embodiment of the present invention will now be described mainly with reference to FIGS. 13 to 15. FIG. 13 is a view showing a configuration of the third embodiment of the present invention. Although the basic configuration of an electrical surgical device 1 is the same as the one described in connection with the first embodiment of FIG. 4, there is a difference that an electric knife 50 to be used for laparoscopy is connected in place of the electric knife 3 for flexible scope.

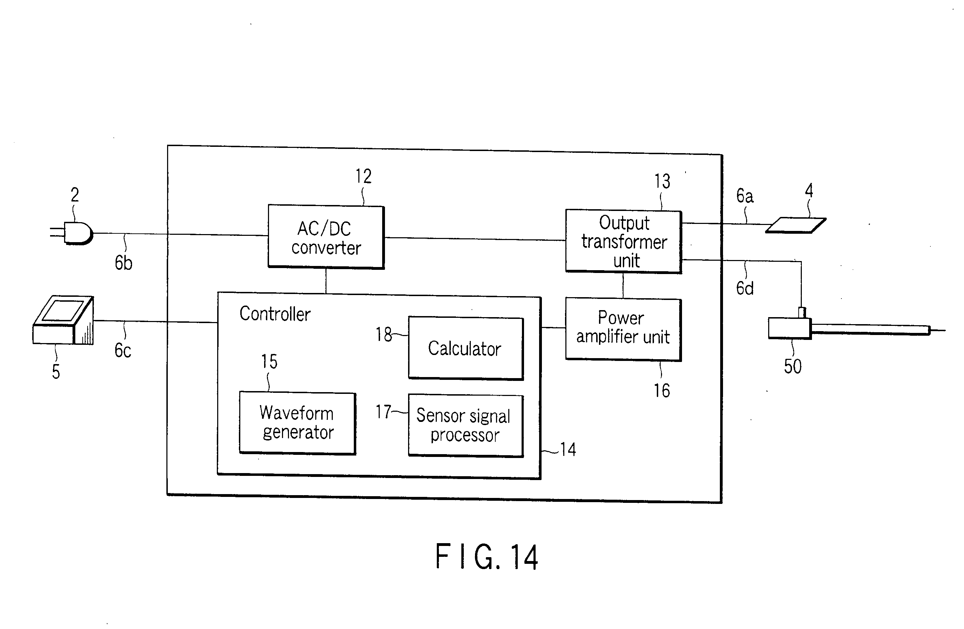

FIG. 14 is a diagram showing an internal configuration of the electrical surgical device 1. The internal configuration is also the same as the one described in connection with the first embodiment of FIG. 4. Since the function of the configuration of the third embodiment is the same as the one shown in the flowchart of FIG. 5, a detailed description thereof is omitted herein.

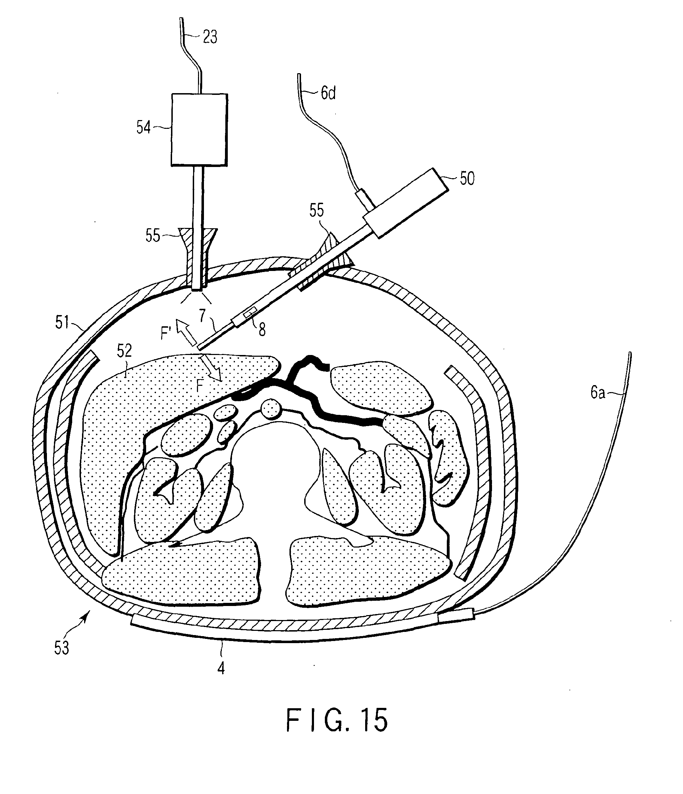

A case where the foregoing third embodiment is applied to an endoscopic surgical operation will now be described in detail with reference to FIG. 15. FIG. 15 is a sectional view of a human abdomen 53 under the endoscopic surgical operation. In the endoscopic surgical operation, the electric knife 50 is combined with a rigid endoscope 54 to perform a treatment.

A case of resection of a liver 52 will now be given as an example of the endoscopic surgical operation, in particular. A plurality of holes are previously bored in the human abdomen 53, and trocars 55 through which the rigid endoscope 54 and a treatment tool are passed are inserted in advance into the holes, individually. The rigid endoscope 54 and electric knife 50 are inserted individually into the trocars 55 when they are actually used. In order to secure a space for the operation, the human abdomen 53 is previously filled with a gas, such as carbon dioxide. Further, a counterelectrode plate 4 is previously affixed to the back.

The operator operates the electric knife 50 so that an electrode section 7 contacts the liver 52. By applying a high-frequency voltage between the electrode section 7 and electrode plate 4 in this state, the operator gradually cauterizes and incises the liver 52. As shown in the drawing, a force F from the electrode section 7 acts on the liver 52, while a repulsion F' from the liver 52 acts on the electrode section 7. Hereupon, a relational expression F = F' holds for the force F and repulsion F'. If the high-frequency voltage is applied with the force F to an excessively high degree, the liver 52 may be incised beyond necessity or perforated.

In the third embodiment described above, the application of the high-frequency voltage to the electrode section 7 is controlled if the repulsion F' detected by a strain gauge 8 is above a threshold value. Thus, if excessive force is applied to the electrode section 7, excessive incision or perforation of the liver 52 can be prevented to improve the safety.

According to the present embodiment, the endoscopic surgical operation is given as an example of the operation. If the principle described in connection with the present embodiment is used, however, excessive incision or perforation of the organism can be prevented to improve the safety when excessive force is applied to the electrode section 7 in any of operations that utilize other surgical devices. In this case, the present embodiment may be used for laparotomic surgery without being specially combined with an endoscope.

The following is a description of the correspondence between the individual devices of the present embodiment and the processing sections shown in FIG. 1. A foot switch 5 corresponds to the energy instruction input section 105. A controller 14 corresponds to the regulation section 104. An output transformer unit 13 corresponds to the energy supply section 103. The electric knife 50 corresponds to the treatment section 101. The strain gauge 8 corresponds to the state-change detecting section 102.

Although the electrode section 7 used according to the present embodiment is of a needle type, its shape is not specially limited. An unbent or insertable shape may be used instead.

### (Fourth Embodiment)

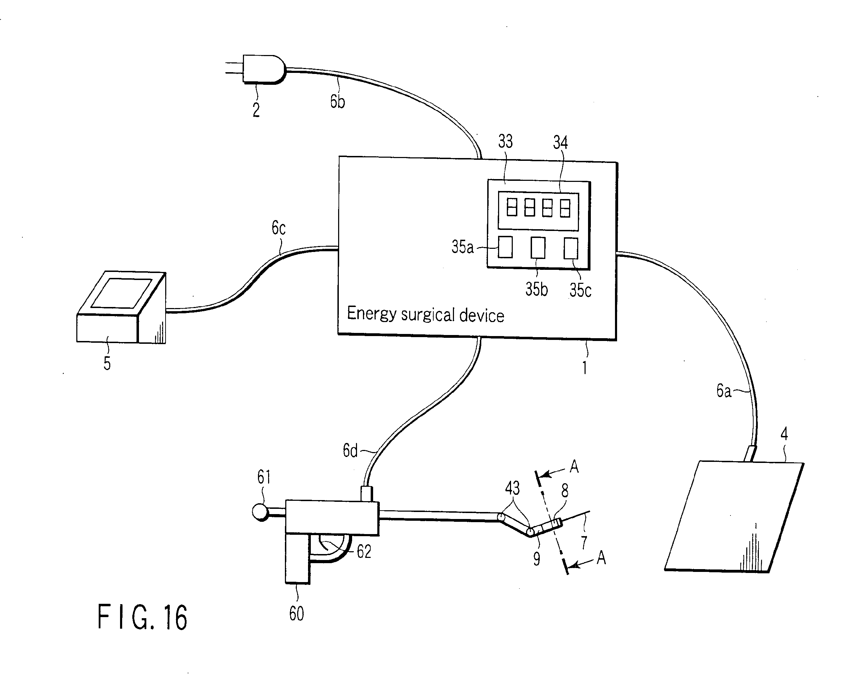

A fourth embodiment of the present invention will now be described mainly with reference to FIGS. 16 to 18.

FIG. 16 is a view showing a configuration of the fourth embodiment of the present invention. Although the basic configuration of an electrical surgical device 1 is the same as the one described in connection with the second embodiment of FIG. 9, there is a difference that an electrically-driven bendable knife 60 to be used for laparoscopy is connected in place of the electrically-driven bendable knife 40 for flexible scope. Further, a joystick 61 and lever 62 are used in place of the bending instruction input unit 41 as means for instructing the bending sections 43 to bend. Furthermore, an electromagnetic motor, pulley, and wire, none of which is shown, are supposed to be incorporated in the electrically-driven bendable knife 60.

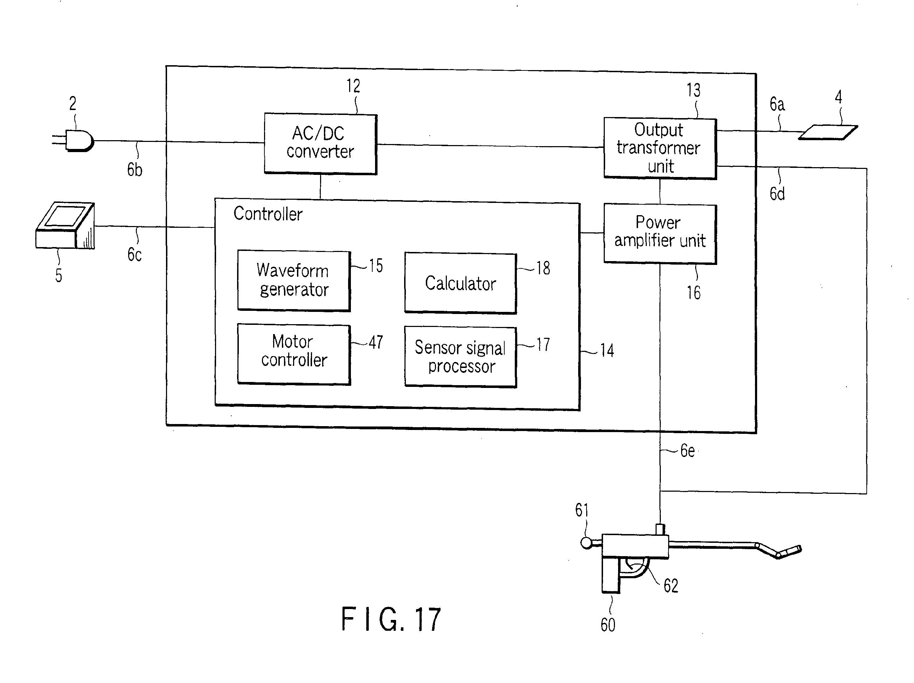

FIG. 17 is a diagram showing an internal configuration of the electrical surgical device 1. The internal configuration is also the same as the one described in connection with the second embodiment of FIG. 11. The function of the configuration of the fourth embodiment is the same as the one shown in the flowchart of FIG. 12.

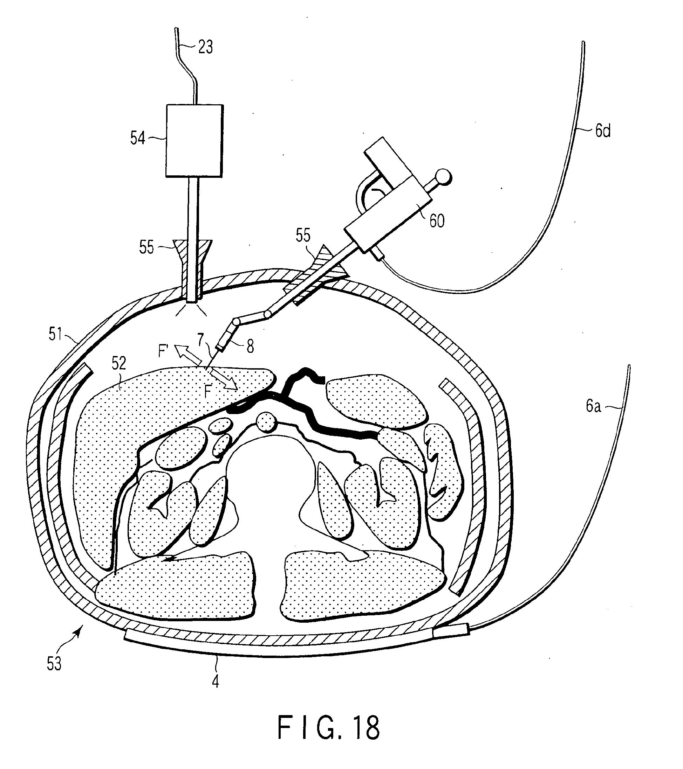

A case where the foregoing fourth embodiment is applied to an endoscopic surgical operation will now be described in detail with reference to FIG. 18. FIG. 18 is a sectional view of a human abdomen 53 under the endoscopic surgical operation. In the endoscopic surgical operation, the electrically-driven bendable knife 60 is combined with a rigid endoscope 54 to perform a treatment.

A case of resection of a liver 52 will now be given as an example of the endoscopic surgical operation, in particular.

A plurality of holes are previously bored in the human abdomen 53, and trocars 55 through which the rigid endoscope 54 and a treatment tool are passed are inserted in advance into the holes, individually. The rigid endoscope 54 and electrically-driven bendable knife 60 are inserted individually into the trocars 55 when they are actually used. In order to secure a space for the operation, the human abdomen 53 is previously filled with a gas, such as carbon dioxide. Further, a counterelectrode plate 4 is previously affixed to the back.

The operator operates the electrically-driven bendable knife 60 so that the shape of the bending sections 43 is changed to settle an optimum position for the resection and an electrode section 7 then contacts the liver 52. By applying a high-frequency voltage between the electrode section 7 and counterelectrode plate 4 in this state, the operator gradually cauterizes and resects the liver 52. As shown in the drawing, a force F from the electrode section 7 acts on the liver 52, while a repulsion F' from the liver 52 acts on the electrode section 7. Hereupon, a relational expression F = F' holds for the force F and repulsion F'. If the high-frequency voltage is applied with the force F to an excessively high degree, the liver 52 may be incised beyond necessity or perforated.

In the fourth embodiment described above, the bending of the bending sections 43 is controlled so that F is reduced if the repulsion F' detected by a strain gauge 8 is above a threshold value. If F is above the threshold value after the bending control, the high-frequency voltage applied to the electrode section 7 is controlled. Thus, if excessive force is applied to the electrode section 7, excessive incision or perforation of the liver 52 can be prevented to improve the safety. According to the present embodiment, the endoscopic surgical operation is given as an example of the operation. If the principle described in connection with the present embodiment is used, however, excessive incision or perforation of the organism can be prevented to improve the safety when excessive force is applied to the electrode section 7 in any of operations that utilize other energy surgical devices. In this case, the present embodiment may be used for laparotomic surgery without being specially combined with an endoscope.

The following is a description of the correspondence between the individual devices of the present embodiment and the processing sections shown in FIG. 8. The joystick 61 and lever 62 correspond to the power instruction input section 126. A foot switch 5 corresponds to the energy instruction input section 127. A controller 14 corresponds to the control section 125-1 and regulation section 125-2. An output transformer unit 13 corresponds to the energy supply section 124. The electrically-driven bendable knife 60 corresponds to the power section 121 and treatment section 122. The strain gauge 8 corresponds to the state-change detecting section 123.

Although the wire and electromagnetic motor are used as means for bending the bending sections 43, they may be successfully replaced with any other means that include a bending mechanism. For example, a pneumatic actuator may be substituted for the electromagnetic motor, and an artificial muscle may be used in place of the wire and electromagnetic motor.

Although the smoothly bendable bending sections 43 are used as means for controlling the position of the electrode section 7 according to the present embodiment, moreover, it may be replaced with any means that can change the position of the electrode section 7. For example, an angling mechanism or a rotation mechanism may be used instead.

Although the joystick 61 and lever 62 are used as the bending instruction section according to the present embodiment, furthermore, the form of an input method is not specially limited in the present embodiment. For example, a haptic device, a touch-panel monitor, or voice recognition may be used instead.

Although the bending instruction section is incorporated in the electrically-driven bendable knife 60 according to the present embodiment, moreover, it may alternatively be kept away from the electrically-driven bendable knife.

Although the bending of the bending sections 43 and the high-frequency signal are controlled according to the present embodiment, furthermore, only one of them may be controlled. In the case where only one is controlled, an input interface may be provided that allows the operator to select the controlled object.

Although the electrode section 7 used according to the present embodiment is of a needle type, its shape is not specially limited. An unbent or insertable shape may be used instead.

Although the amount of force that acts on the electrode section 7 is given as an example of a state variation measured by the state-change detecting section according to the first to fourth embodiments described above, furthermore, the state variation to be measured is not limited to the amount of force. For example, a strain that acts on the electrode section 7, temperature variation, velocity, acceleration, or position information may be measured instead.

Although the strain gauge 8 is used as a sensing section for the force that acts on the electric knife 3 for flexible scope according to each of the foregoing embodiments, moreover, it may be replaced with another type of force detecting sensor, such as an optical fiber, piezoelectric-sensor, semiconductor strain gauge, or capacitance sensor. Further, a temperature sensor, MEMS pressure sensor, gyro-sensor, or magnetic sensor may be substituted for the force detection.

Furthermore, the method of affixing the strain gauge 8 is not specially limited. As shown in FIGS. 7A and 7B, three or two strain gauges 8 may be used. If this is done, the direction and accuracy of measurement of the force that acts on the electrode section 7 are damaged. However, the strain gauges 8 and cables that connect these strain gauges 8 can be reduced in number, so that the device can be simplified and reduced in cost.

Further, the strain gauge 8 may be affixed in another position where the force on the electrode section 7 can be measured. Although the foot switch 5 is used as the instruction input section for high-frequency energy, moreover, it may be replaced with another instruction input section, such as a hand switch or a voice recognition switch.

Although the SUS304 is used as the material of the electrode section 7, moreover, it may be replaced with any other electrically-conductive substance.

In each of the foregoing embodiments, furthermore, the high-frequency signal of the electrode section 7 is on-off controlled based on determination whether or not the sensing information obtained in the sensor signal processor 17 exceeds the threshold value. Alternatively, however, the controller 14 may be configured to regulate the high-frequency signal of the electrode section 7 based on the sensing information obtained in the sensor signal processor 17.

Although the electric knife for flexible scope is used in each of the foregoing embodiments, moreover, it may be applied to some other energy surgical device, such as an ultrasonic treatment tool.

### (Fifth Embodiment)

The following is a description of a fifth embodiment of the present invention. Perforation by an incision tool or the like can be given as an example of an accident during the endoscopic submucosal dissection. This occurs because the incision tool is pressed firmly against a proper muscle layer. In an angle operation of an endoscope, however, it is difficult for the operator to feel a force that acts on an incision section on the distal end of the incision tool that is passed through a channel of the endoscope. In marking a region around a lesion by pressing the incision tool against a mucous membrane, moreover, it is also difficult to feel the force on the incision tool that is long.

In order to solve the above problem, the fifth embodiment is characterized by the following configurations.
1. The device has a function to detect the force that acts on the incision section on the distal end of the incision tool.
2. A manipulator is used to drive the incision tool for feed and distribution, based on item 1.
3. The device is provided with a mechanism that issues a warning before the amount of force that acts on the incision section reaches the amount of force that perforates the proper muscle layer, based on item 1 or 2.
4. A warning portion is displayed on an endoscopic view monitor, based on item 3.
5. The device is provided with a function to stop the operation when the amount of force that acts on the incision section reaches the amount of force applied immediately before the proper muscle layer is perforated, based on item 2.
6. A treatment tool used in a treatment tool system is a local injection needle, based on items 2 to 5.

### (Sixth Embodiment)

The following is a description of a sixth embodiment of the present invention. Bleeding by an incision tool or the like can be given as an example of an accident during the endoscopic submucosal dissection. A high-frequency power unit that has been developed as a concept for hemostatic incision is mounted with an end cut mode for alternate switching between coagulation and cutting modes. If the incision tool is moved fast, it incises a blood vessel before coagulation of blood, so that bleeding may be caused in some cases. Since the bleeding requires a hemostatic operation, moreover, it greatly influences the treatment time.

In order to solve the above problem, the sixth embodiment is characterized by the following configurations.
1. The incision tool used in combination with the high-frequency power unit has a function to electrically control the drive of a knife section on its distal end and control the driving speed of the knife section in association with information on output setting of the high-frequency power unit.
2. In an endoscope through which the incision tool used in combination with the high-frequency power unit is passed and which includes a drive mechanism capable of driving the incision tool in an arbitrary direction, the drive of the treatment tool is electrically controlled, and the driving speed of the knife section is controlled in association with the information on output setting of the high-frequency power unit.
3. The mode of the high-frequency power unit is an end-cut mode, based on item 1 or 2.

According to the first and third embodiments described above, excessive incision or perforation of the organism can be prevented to improve the safety as the controller controls the amount of energy supply to the electrode section based on the signal obtained by the strain gauge.

According to the second and fourth embodiments, moreover, a load that acts on the electrode section is reduced as the controller controls the bending sections in bending based on the signal obtained by the strain gauge. If the load on the electrode section is high despite the control of the bending sections in bending, moreover, the controller controls the electrode section in conduction. Thus, there is provided a device that is improved in safety, since excessive incision or perforation of an organism can be prevented, so that it can be safely employed even by a novice doctor with experience of relatively few surgical cases.

### Industrial Applicability

According to the present invention, the regulation section is provided for regulating the energy supply by the energy supply section or power, based on a result of detection by the state-change detecting section, so that excessive incision or perforation of the organism can be prevented to improve the safety during the operation.

## Claims

1. An energy surgical device comprising:
a treatment section which treats a subject;
an energy supply section which supplies energy to the treatment section;
a state-change detecting section which detects a state change of the treatment section;
an energy instruction input section through which an instruction for the energy supply is input; and
a regulation section which regulates the energy supply by the energy supply section based on the input of the energy supply instruction through the energy instruction input section and a result of detection in the state-change detecting section.

2. An energy surgical device according to claim 1, wherein the regulation section is provided with a threshold input section which inputs a threshold value of the state variation.

3. An energy surgical device according to claim 1, wherein the energy surgical device is used together with an endoscope.

4. An energy surgical device comprising:
a treatment section which treats a subject;
an energy supply section which supplies energy to the treatment section;
a force amount change detecting section which detects a change of an amount of force received by the treatment section;
an energy instruction input section which inputs an instruction for the energy supply; and
a regulation section which regulates the energy supply by the energy supply section based on the input of the energy supply instruction through the energy instruction input section and a result of detection in the state-change detecting section.

5. An energy surgical device according to claim 4, wherein the regulation section is provided with a threshold input section which inputs a threshold value of the force amount variation.

6. An energy surgical device according to claim 4, wherein the energy surgical device is used together with an endoscope.

7. An energy surgical device comprising:
a treatment section which treats a subject;
an energy supply section which supplies energy to the treatment section;
a state-change detecting section which detects a state change of the treatment section;
an energy instruction input section which inputs an instruction for the energy supply;
a power section which actively drives the treatment section;
a power instruction input section which instructs the power section to operate;
a control section which controls the power section in accordance with the operation instruction through the power instruction input section; and
a regulation section which regulates the energy supply by the energy supply section and/or the drive by the power section based on the input of the power instruction through the power instruction input section and a result of detection in the state-change detecting section.

8. An energy surgical device according to claim 7, wherein the state-change detecting section detects a change of an amount of force received by the treatment section.

9. An energy surgical device according to claim 7, wherein the regulation section is provided with a threshold input section which inputs a threshold value of the state variation.

10. An energy surgical device according to claim 9, wherein the regulation section regulates the drive of the power section lest the state variation of the state-change detecting section exceed the threshold value.

11. An energy surgical device according to claim 9, wherein the regulation section regulates the energy supply by the energy supply section when the state variation of the state-change detecting section exceeds the threshold value.

12. An energy surgical device according to claim 7, wherein the energy surgical device is used together with an endoscope.
